(19) Europäisches Patentamt
European Patent Office
Office européen des brevets

(11) Numéro de publication : **0 346 165 B1**

(12) # FASCICULE DE BREVET EUROPEEN

(45) Date de publication du fascicule du brevet : **27.05.92 Bulletin 92/22**

(51) Int. Cl.$^5$ : **C08F 20/36,** C09K 19/58

(21) Numéro de dépôt : **89401167.5**

(22) Date de dépôt : **25.04.89**

(54) **Polymères mésomorphes à chaînes latérales comportant un motif trans (phénylène substitué en position 4-4')-2 dioxanne 1,3alkényl-5.**

(30) Priorité : **06.05.88 FR 8806118**

(43) Date de publication de la demande : **13.12.89 Bulletin 89/50**

(45) Mention de la délivrance du brevet : **27.05.92 Bulletin 92/22**

(84) Etats contractants désignés : **CH DE FR LI**

(56) Documents cités :
**GB-A- 2 002 767**
**DIE MAKROMOLEKULARE CHEMIE, vol. 188, 1987, pages 1017-1031, Bâle, CH; C.S.HSU et al.: "Liquid crystalline polymerscontaining heterocycloalkanediyl groups as mesogens, 1 Liquid crystalline polymethacrylates and polyacrylates containing1,3-dioxane-2,5-diyl groups as mesogens in the side chain"**
**CHEMICAL ABSTRACTS, vol. 105, no. 26, 29 décembre 1986, page 6, no. 227428f, Columbus, Ohio, US; C.S.HSU et al.: "Side-chainliquid-crystalline polymethacrylates and polycrylates containing 2,5-disubstituded-1,3-dioxane mesogens"**

(73) Titulaire : **THOMSON RECHERCHE**
**173, bld Haussmann**
**F-75008 Paris (FR)**

(72) Inventeur : **Le Barny, Pierre**
**THOMSON-CSF SCPI-Cédex 67**
**92045 Paris la Défense (FR)**
Inventeur : **Leborgne, Alain**
**THOMSON-CSF SCPI-Cédex 67**
**92045 Paris la Défense (FR)**
Inventeur : **Legrand, Cécile**
**THOMSON-CSF SCPI-Cédex 67**
**92045 Paris la Défense (FR)**
Inventeur : **Noel, Claudine**
**THOMSON-CSF SCPI-Cédex 67**
**92045 Paris la Défense (FR)**
Inventeur : **Vairon, Jean-Pierre**
**THOMSON-CSF SCPI-Cédex 67**
**92045 Paris la Défense (FR)**

(74) Mandataire : **Guérin, Michel et al**
**THOMSON-CSF SCPI 51, Esplanade du Général de Gaulle**
**F-92045 PARIS LA DEFENSE CEDEX 67 (FR)**

EP 0 346 165 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne une nouvelle famille de polymères cristaux liquides à température de transition vitreuse élevée (supérieure à 50°C) pouvant présenter une phase nématique et/ou une phase smectique. Ces nouveaux polymères sont utilisables en optique non linéaire sous forme de couche dopée dans des systèmes dits "guest-host" à orientation gelée thermiquement.

A cause de leur facilité de mise en oeuvre, les films de polymère dopés avec des molécules ayant une forte hyperpolarisabilité β (système guest-host) puis polarisés au moyen d'un champ électrique continu, présentent un grand intérêt en optique non linéaire et plus particulièrement dans les effets du second ordre. Deux types de polymères peuvent être utilisés comme host dans les systèmes guest-host : les polymères amorphes comme le polyméthacrylate de méthyle (PMMA) et les polymères cristaux liquides à chaînes latérales. Des travaux récents ont démontré que la valeur du plus grand coefficient ($d_{33}$)du tenseur de susceptibilité d'ordre 2 ( $\chi^{(2)}$) est plus élevée lorsqu'on utilise un polymère cristal liquide à la place d'un polymère amorphe dans les systèmes guest-host. Ce coefficient $d_{33}$ peut alors être multiplié par un facteur compris entre 1 et 5 (voir K.D. SINGER, M.G. KUZYK, J.E. SOHN, JOURNAL OF THE OPTICAL SOCIETY OF AMERICA, B. Vol. 4, n° 6 , 1987).

Cependant, les systèmes de type "guest-host" formés avec des polymères cristaux liquides à chaînes latérales sont le siège après polarisation, de phénomènes de relaxation qui ont pour effet de diminuer la polarisation du matériau, ce qui a pour conséquence de provoquer une perte d'efficacité dans la conversion de l'onde de pulsation ω en onde de pulsation 2ω. Le phénomène de relaxation observé est principalement dû au fait que les polymères mésomorphes à chaînes latérales ont une température de transition vitreuse basse (proche de la température ambiante).

Afin de pallier ces inconvénients, l'invention propose une famille d'homopolymères du type polyacrylate dérivant du trans (phénylène substitué en position 4-4′)-2 dioxanne 1,3 alkényl-5 et qui possèdent des températures de transition vitreuse élevées par rapport à la température ambiante. Ces polymères peuvent présenter une phase nématique et éventuellement une phase smectique.

L'invention a donc pour objet un polymère mésomorphe à chaînes latérales comportant un motif 1,3 dioxanne, caractérisé en ce que le polymère répond à la formule générale suivante:

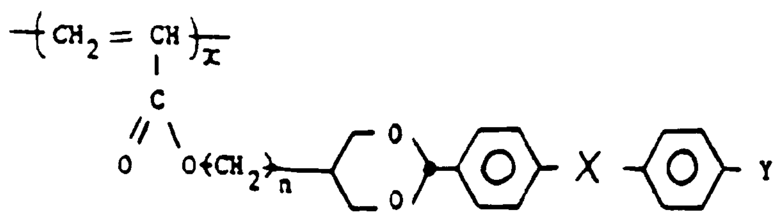

x indiquant le degré de polymérisation,
X étant

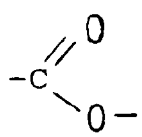

ou

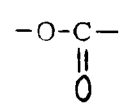

Y étant CN, $CF_3$ ou $OCH_3$
et $2 \leqq n \leqq 12$.

L'invention a aussi pour objet un procédé de fabrication des polymères mésomorphes décrits plus haut,caractérisé en ce qu'il comprend les étapes suivantes :

– 1ère étape : obtention du bromo-ω alcanol par réduction de l'acide bromo-ω alcanoïque correspondant,
– 2ème étape : synthèse du (tétrahydro-2 pyranoxy) - α (bromo)-ω alcane par réaction du bromo-ω alcool obtenu à l'issue de la 1ère étape, avec du dihydropyranne en présence de résine sulfonique,
– 3ème étape : obtention du (tétrahydro-2 pyranoxy-ω alkyl) malonate d'éthyle par réaction entre le produit obtenu à l'issue de la 2è étape et le sodiomalonate d'éthyle,
– 4ème étape : synthèse du (tétrahydro-2 pyranoxy)- ω (hydroxyméthyl)- 2 alcanol par réduction du produit obtenu à l'issue de la 3ème étape,
– 5ème étape : synthèse de l'hydroxyméthyl-2 alcane diol α, ω par déprotection du groupement hydroxyle

du produit obtenu à l'issue de la 4ème étape,

– 6ème étape : synthèse des composés de formule générale

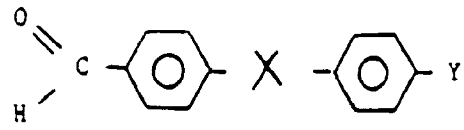

par estérification :

. soit entre le carboxy -4 benzaldéhyde et le phénol substitué en position 4 par le radical Y, lorsque X est

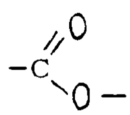

. soit entre l'hydroxy-4 benzaldéhyde et l'acide benzoïque substitué en position 4 par le radical Y, lorsque X est

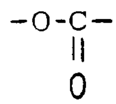

– 7ème étape : synthèse du composé de formule générale

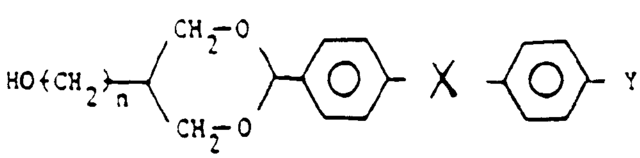

par acétalisation du composé synthétisé à la 6ème étape par l'hydroxyméthyl-2 alcane diol , obtenu à l'issue de la 5ème étape,

– 8ème étape : synthèse du monomère de formule générale :

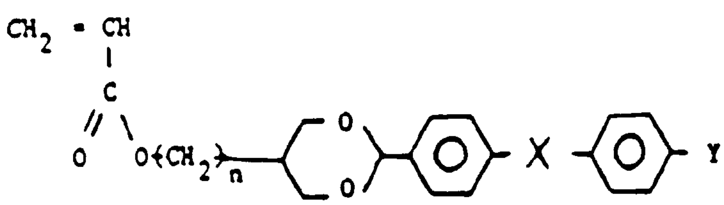

par estérification du composé synthétisé à la 7ème étape à l'aide du chlorure d'acryloyle,

– 9ème étape : polymérisation du monomère obtenu à l'issue de la 8ème étape.

L'invention a encore pour objet l'utilisation de ces polymères en optique non linéaire.

La description qui va suivre portera sur le procédé général de synthèse des polymères selon l'invention ainsi que sur les caractéristiques physiques de ces polymères.

A ce jour, un nombre limité de polymères cristaux liquides comportant le motif 1,3 dioxanne dans leurs chaînes latérales ont été synthétisés. Ce sont essentiellement des polyacrylates, des polyméthacrylates et des polysiloxannes formés à partir du groupement mésogène trans (méthoxy-4 phényl) -5 phényl -2 dioxanne 1,3 (C.S. HSU, J.M. RODRIGUEZ-PARADA, V. PERCEC, MAKROMOLECULAR CHEMIE, 188, P. 1017-1031, 1987) ou des polysiloxannes formés à partir des groupements mésogènes suivants : trans (méthoxy-4 phényl)-2 dioxanne 1,3 et trans (méthoxy-4 phényl)-5 dioxanne 1,3 (C.S. HSU, V. PERCEC, POLYMER BULLETIN

17, P. 49-54, 1987). Ces composés sont caractérisés par une faible anisotropie diélectrique.

Quelques polymères cristaux liquides ayant une anisotropie diélectrique positive importante ont été synthétisés. Ce sont des polysiloxannes portant le groupement mésogène trans (cyano-4 phényl)-2 dioxanne 1,3 yl-5 (C.S. HSU, V. PERCEC, MAKROMOLECULAR CHEMIE, RAPID COMMUNICATION 8, P 331-337, 1987). Ces polymères sont caractérisés par une température de transition vitreuse très basse (inférieure à -100°C) ce qui les rend inutilisables en optique non linéaire.

Procédé général de synthèse.

Les polymères selon l'invention s'obtiennent par polymérisation radicalaire des monomères correspondants.

Les monomères sont obtenus à partir des trois unités suivantes :

$CH_2 = CH - COCl$ (A)

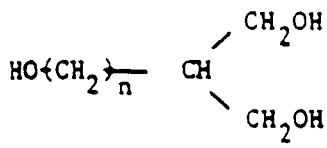

(B)

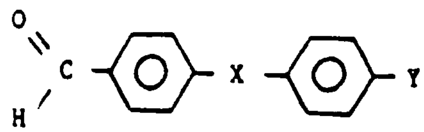

(C)

Les unités (B) et (C) ont été synthétisées. Les produits commerciaux suivants ont été employés :

- le chlorure d'acryloyle,
- l'acide bromo-ω alcanoïque,
- le dihydropyranne,
- le malonate d'éthyle,
- l'hydroxy-4 benzaldéhyde,
- l'acide cyano-4 benzoïque,
- l'acide trifluorométhyl-4 benzoïque,
- l'acide méthoxy-4 benzoïque,
- le carboxy-4 benzaldéhyde,
- le cyano-4 phénol,
- le méthoxy-4 phénol,
- le trifluorométhyl-4 phénol.

Réaction 1: Obtention du bromo-ω alcanol.

Cet alcool est obtenu par réduction de l'acide bromo-ω alcanoïque correspondant au moyen de l'hydrure de lithium et d'aluminium dans l'éther.

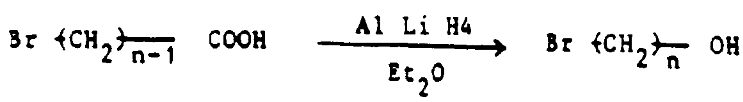


Dans le cas où n = 6, le point d'ébullition du liquide obtenu est de 101°C, sous une pression de 4 mm de mercure et le rendement de la réaction est de 70 %.

Les alcools correspondant à n = 2, 3 et 11 sont disponibles dans le commerce.

Réaction 2 : synthèse du (tétrahydro-2 pyranoxy) -α(bromo)-ω alcane.

La fonction alcool est protégée par réaction du bromo-ω alcool (obtenu à l'issue de la réaction 1) avec du dihydropyranne (DHP) en présence de résine sulfonique du type de la marque déposée Amberlyst 15.

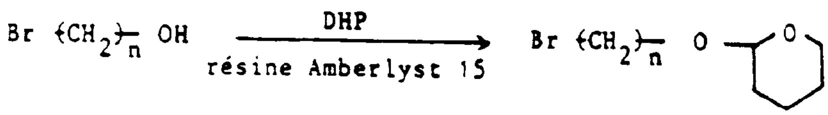


Le rendement est quantitatif.

Réaction 3: Obtention du (tétrahydro-2 pyranoxy-ω alkyl) malonate d'éthyle.

Ce composé est préparé par réaction entre le (tétrahydro-2 pyranoxy)-α(bromo)-ω alcane (obtenu à l'issue de la réaction 2) et le sodiomalonate d'éthyle obtenu par réaction entre le malonate d'éthyle et l'éthanolate de sodium en solution dans l'éthanol absolu.

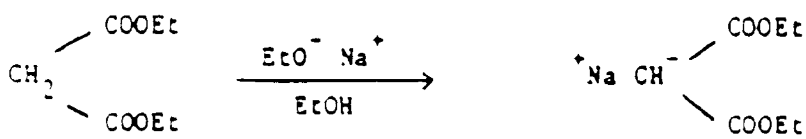


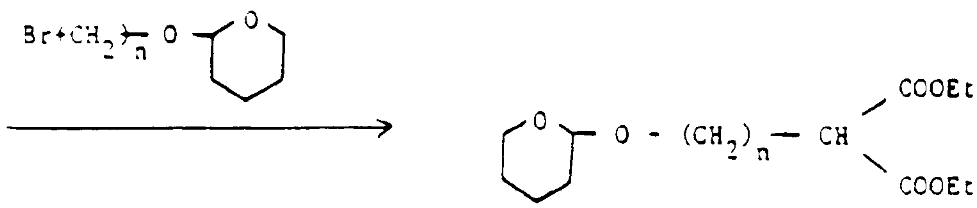

Lorsque n = 6, par exemple, le produit est purifié par chromatographie sur colonne de silice (Kieselgel 60). L'éluant est un mélange acétate d'éthyle (10 % en volume) / toluène (90 % en volume). Le rendement est de 80 %.

Réaction 4 : Synthèse du (tétrahydro-2 pyranoxy)-ω (hydroxyméthyl)-2 alcanol.

Ce composé est obtenu par réduction du dérivé précédent à l'aide d'hydrure de lithium et d'aluminium dans l'éther.

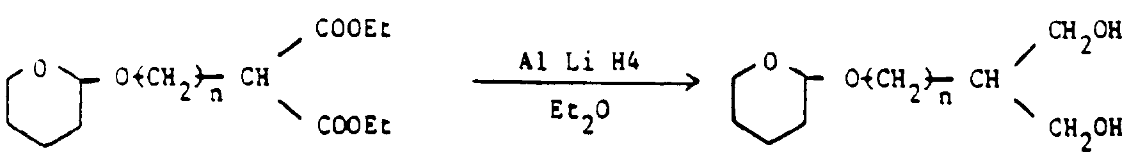


Lorsque n = 6, par exemple, le rendement de la réaction est de 90 %.

Ce composé est utilisé sans purification particulière dans la réaction suivante.

Réaction 5 : Synthèse de l'hydroxyméthyl-2 alcane diol α,ω (triol B).

Le groupement hydroxyle est déprotégé en utilisant la résine sulfonique Amberlyst 15 en milieu méthanol.

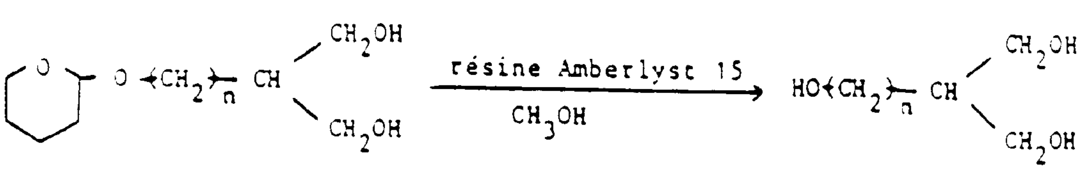


C'est l'unité (B).

Pour n = 6, le rendement est de 90 %. Le triol est purifié par recristallisation dans un mélange n-hexane 50 % / acétone 50 % en volume. Son point de fusion est de 50°C.

Réaction 6 : Synthèse des composés de formule générale

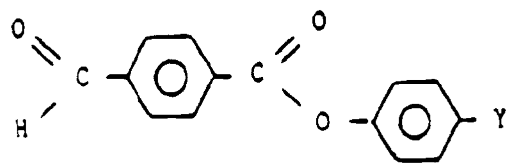

avec Y = CN, $CF_3$ ou $OCH_3$.

Ces composés sont obtenus par estérification entre le carboxy-4 benzaldéhyde et le phénol substitué en position 4 (CN, $CF_3$, $OCH_3$) en utilisant le dicyclohexyl-1,3 carbodiimide (DCCI) comme agent d'estérification en présence de diméthylamino-4 pyridine (DMAP) comme catalyseur dans le chlorure de méthylène.

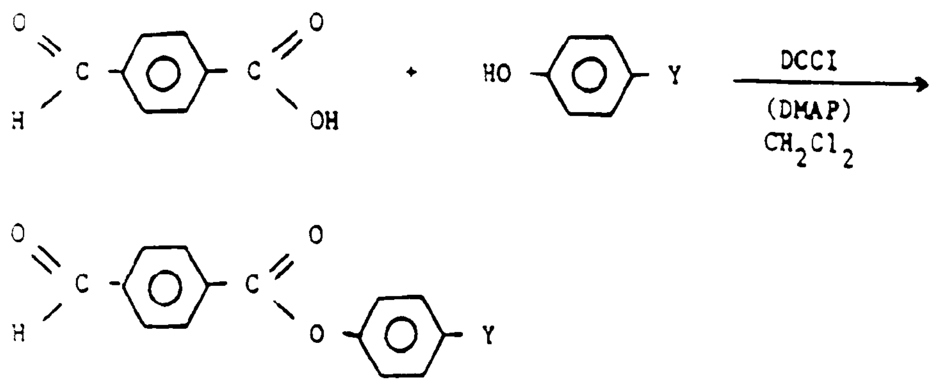


Lorsque Y = $OCH_3$, le rendement est de 95 %. Le point de fusion du produit obtenu est de 102°C.

Réaction 7 : Synthèse des composés de formule générale

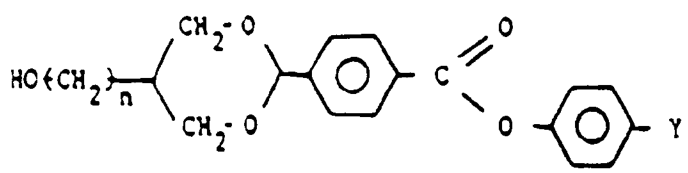

(cis + trans)

Ces composés sont préparés par acétalisation des dérivés synthétisés précédemment par l'hydroxyméthyl-2 alcane diol, (obtenu à l'issue de la réaction 5) dans le tétrahydrofuranne en présence de résine sulfonique Amberlyst 15 et de tamis moléculaire 4 angströms (selon la méthode décrite par J.C. MESLARD, F. SUBIRA, J.P. VAIRON, A. GUY et R. GARREAU dans le BULLETIN DE LA SOCIETE CHIMIQUE FRANCAISE, 1985 (1), p. 84).

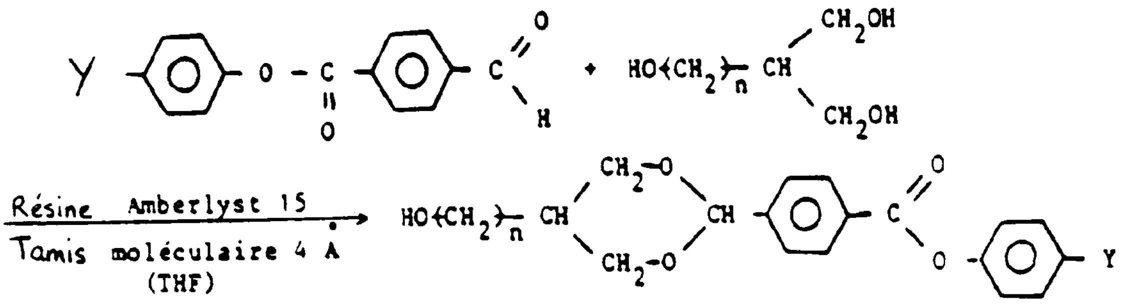


Y = $OCH_3$, CN, $CF_3$.

Par exemple, lorsque n = 6 et Y = $OCH_3$, le rendement est quantitatif. Un mélange d'isomères (cis + trans) est obtenu.

Réaction 8 : Synthèse des monomères de formule générale

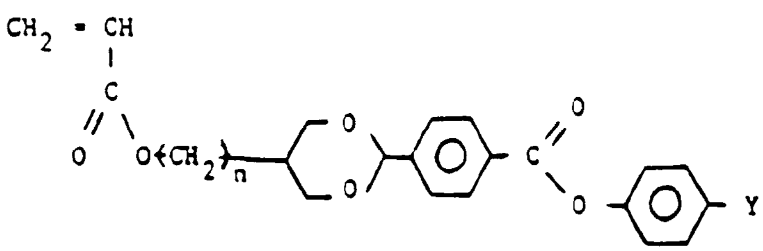

Ces monomères sont obtenus par estérification des dérivés obtenus à l'issue de la réaction 7 à l'aide de chlorure d'acryloyle dans le chlorure de méthylène en présence de triéthylamine.

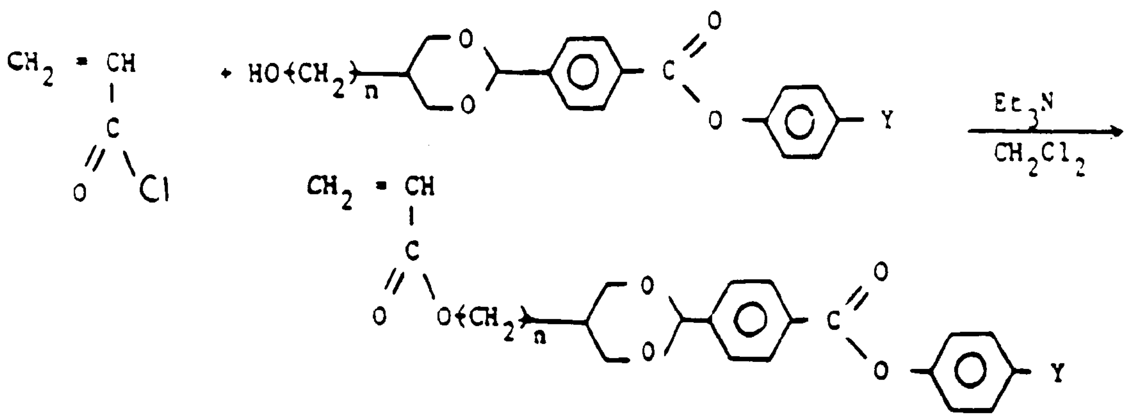


(cis + trans)

Lorsque n = 6 et que Y est -$OCH_3$, le mélange d'isomères cis + trans est recristallisé dans un mélange comprenant 80 % d'hexane et 20 % d'acétate d'éthyle (proportions volumiques). L'isomère trans est obtenu avec un rendement de 65 %. La résonance magnétique nucléaire (sur protons) permet de vérifier la pureté de ce composé (absence d'isomère cis). Ce monomère présente le comportement thermique suivant :

K 97°C N 161°C L

K désignant la phase cristalline, N la phase nématique et L la phase liquide.

L'isomère trans peut être représenté de la manière suivante :

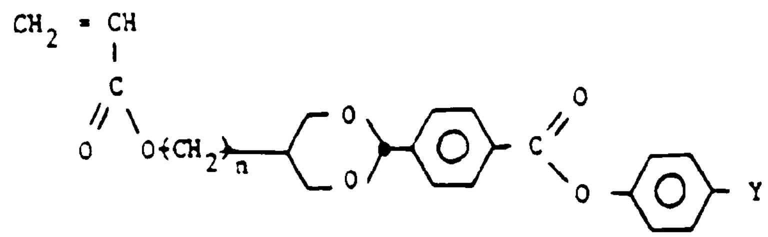

Réaction 9 : Polymérisation des monomères.

La polymérisation peut être effectuée sous vide en utilisant l'α, α' azobisisobutyronitrile (AIBN) comme amorceur radicalaire et le diméthylformamide comme solvant. Le rapport monomère / amorceur a été pris égal à 200.

Lorsque n = 6 et que Y est $-OCH_3$, le rendement en polymère est de 78 % (durée de la polymérisation : 2 jours à 65°C). La masse moléculaire moyenne en nombre est de 8000, celle en poids est de 13000. Les masses moléculaires ont été déterminées par chromatographie par perméation de gel (GPC) et correspondent à des équivalents polystyrène.

Les monomères de formule générale :

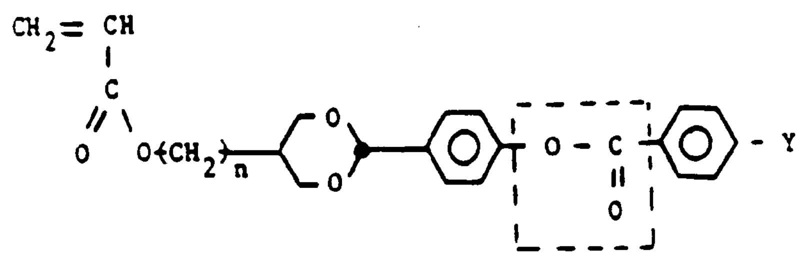

où le mode d'enchaînement des deux noyaux aromatiques est en sens inverse de celui des monomères décrits dans la réaction 8 ont été synthétisés à partir des précurseurs de formule :

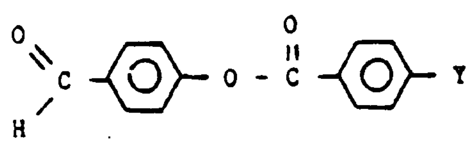

avec Y = $OCH_3$, $CF_3$ ou CN.

Ceux-ci ont été synthétisés à partir de l'hydroxy-4 benzaldéhyde et des acides benzoïques substitués en position 4 par le groupement Y selon la méthode d'estérification décrite pour la réaction 6.

A titre d'exemples :

- pour n = 6 et Y = $OCH_3$, le monomère a le comportement thermique suivant
K 107°C N 168°C L
- pour n = 6 et Y = $CF_3$, le monomère a le comportement thermique suivant
$K_1$ 104°C $K_2$ 115°C $S_A$ 131°C L
$K_1$ et $K_2$ désignant deux phases cristallines de structures différentes et $S_A$ une phase smectique A.

Propriétés physiques des polymères

Les propriétés mésomorphes des polymères préparés ont été étudiées par analyse enthalpique différentielle, microscopie optique et diffraction des rayons X.

A titre d'exemples :

- quand n = 6,

X = 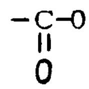

et Y = $OCH_3$, le polymère présente le comportement thermique suivant
G 54°C $N_{re}$136°C S 212°C N 232°C L
les lettres G, $N_{re}$, S, N et L désignant respectivement les phases vitreuse, nématique réentrante, smectique (fluide lamellaire), nématique et liquide.
- quand n = 6,

X = 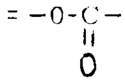

et Y = $OCH_3$, le polymère a le comportement thermique suivant
G 38°C N 238°C L
- quand n = 6,

X = 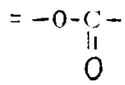

et Y = $CF_3$, le polymère est semi-cristallin. Il fond à 132°C et devient $S_A$ jusqu'à sa température de clarification qui est de 230°C.

Ces exemples sont donnés à titre non limitatif et l'invention concerne notamment tous les composés pour lesquels n varie de 2 à 12.

Les polymères selon l'invention qui possèdent une phase nématique (n = 6, X = -COO- et Y = $OCH_3$ par exemple) et qui ont une température de transition vitreuse supérieure à la température ambiante sont avantageusement utilisables en optique non linéaire et plus particulièrement dans les effets du second ordre comme la génération de seconde harmonique.

## Revendications

1. Polymère mésomorphe à chaînes latérales comportant un motif 1,3 dioxanne, caractérisé en ce que le polymère répond à la formule générale suivante :

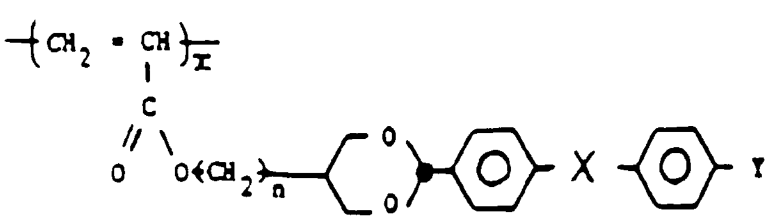

x indiquant le degré de polymérisation,
X étant

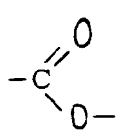 ou 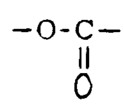

Y étant CN, $CF_3$ ou $OCH_3$
et $2 \leqq n \leqq 12$.

2. Polymère mésomorphe selon la revendication 1, caractérisé en ce que n vaut 6, X est

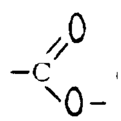

et Y = $OCH_3$.

3. Polymère mésomorphe selon la revendication 1, caractérisé en ce que n vaut 6, X est

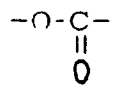

et Y = $OCH_3$.

4. Polymère mésomorphe selon la revendication 1, caractérisé en ce que n vaut 6, X est

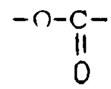

et Y = $CF_3$.

5. Procédé de fabrication de polymères mésomorphe selon l'une quelconque des revendications 1 à 4, caractérisé en ce qu'il comprend les étapes suivantes :

– 1ère étape : obtention du bromo-ω alcanol par réduction de l'acide bromo-ω alcanoïque correspondant,
– 2ème étape : synthèse du (tétrahydro-2 pyranoxy) -α (bromo)-ω alcane par réaction du bromo-ω alcool obtenu à l'issue de la 1ère étape, avec du dihydropyranne en présence de résine sulfonique,
– 3ème étape : obtention au (tétrahydro-2 pyranoxy-ω alkyl) malonate d'éthyle par réaction entre le produit obtenu à l'issue de la 2è étape et le sodiomalonate d'éthyle,
– 4ème étape : synthèse du (tétrahydro-2 pyranoxy)-ω (hydroxyméthyl)- 2 alcanol par réduction du produit obtenu à l'issue de la 3ème étape,
– 5ème étape : synthèse de l'hydroxyméthyl-2 alcane diol α, ω par déprotection du groupement hydroxyle du produit obtenu à l'issue de la 4ème étape,
– 6ème étape : synthèse des composés de formule générale

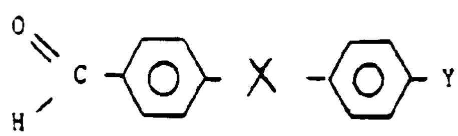

par estérification :

. soit entre le carboxy -4 benzaldéhyde et le phénol substitué en position 4 par le radical Y, lorsque X est

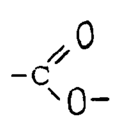

. soit entre l'hydroxy-4 benzaldéhyde et l'acide benzoïque substitué en position 4 par le radical Y, lorsque X est

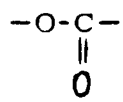

– 7ème étape : synthèse du composé de formule générale

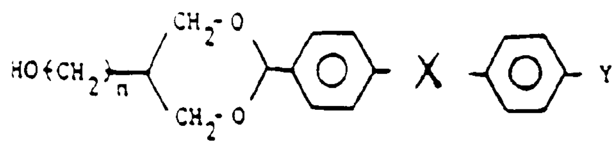

par acétalisation du composé synthétisé à la 6ème étape par l'hydroxyméthyl-2 alcane diol $\alpha,\omega$ obtenu à l'issue de la 5ème étape,

– 8ème étape : synthèse du monomère de formule générale :

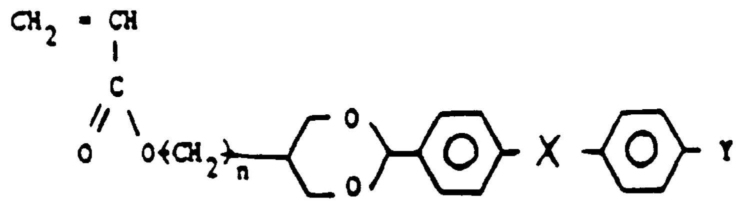

par estérification du composé synthétisé à la 7ème étape à l'aide du chlorure d'acryloyle,

– 9ème étape : polymérisation de l'isomère trans du monomère obtenu à l'issue de la 8ème étape.

## Claims

1. A mesomorphic polymer having side chains comprising a 1,3 dioxane structure, characterized in that the polymer corresponds to the following general formula:

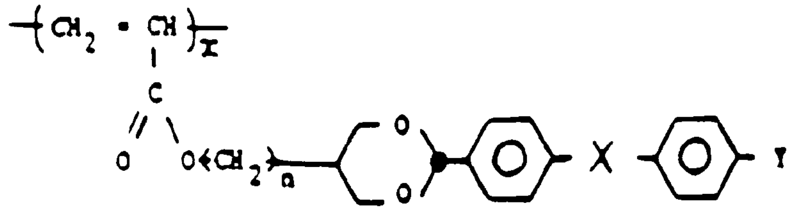

wherein:
X denotes the degree of polymerization,
X is

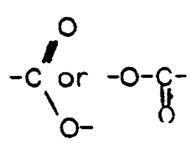
or

Y is CN, $CF_3$ or $OCH_3$
and $2 \leqq n \leqq 12$.

2. The mesomorphic polymer as claimed in claim 1, characterized in that n is equal to 6, X is

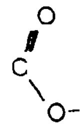

and Y = $OCH_3$.

3. The mesomorphic polymer as claimed in claim 1, characterized in that n is equal to 6, X is

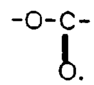

and Y = $OCH_3$.

4. The mesomorphic polymer as claimed in claim 1, characterized in that n is equal to 6, X is

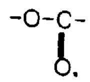

and Y = $CF_3$.

5. A method for the production of mesomorphic polymers as claimed in any one of the preceding claims 1 through 4, characterized in that it comprises the following steps:

– 1st stage: the production of ω-bromoalkanol by the reduction of the corresponding ω-bromoalkanoic acid,

– 2nd stage: the synthesis of ω-(2-tetrahydro-2-pyranoxy)-ω-(bromo)-alkane by the reaction of the ω-bromoalcohol produced at the end of the first stage, with dihydropyrane in the presence of a sulfonic resin,

– 3rd stage: the production of ethyl (2-tetrahydro-ω-pyranoxyalkyl)-malonate by reaction of the product obtained at the end of the second stage and ethyl sodium malonate,

– 4th stage: the synthesis of ω-(2-tetrahydropyranoxy)-2-hydroxymethylalkanol by the reduction of the product obtained at the end of the third stage,

– 5th stage: the synthesis of (2-hydroxymethyl)-α,ω,-alkanediol by the deprotection of the hydroxyl group of the product obtained at the end of the 4th stage, and

– 6th stage: the synthesis of compounds of the general formula:

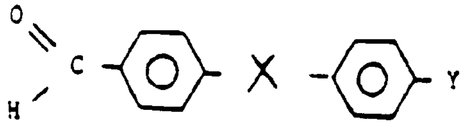

by esterification:

. either between 4-carboxybenzaldehyde and phenol substituted in 4-position by the radical Y, if X is

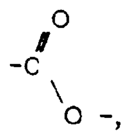

. or between 4-hydroxybenzaldehyde and benzoic acid substituted in 4-position by the radical Y, if X is

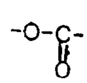

– 7th stage: synthesis of the compound of the general formula

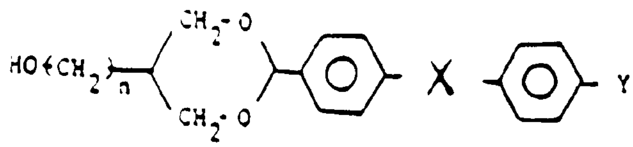

by acetalization of the compound synthesized in the sixth stage by 2-hydroxymethyl-α,ω-alkanediol obtained at the end of the fifth stage,

– 8th stage: the synthesis of a monomer of the general formula

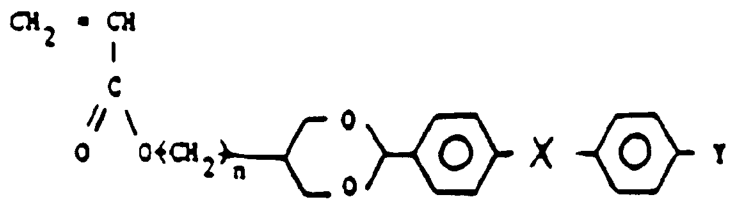

by esterification of the compound synthesized in the seventh stage with the aid of acryloyl chloride, and

– 9th stage: the polymerization of the trans isomer of the monomer obtained at the end of the 8th stage.

**Patentansprüche**

1. Mesomorphes Polymer mit Seitenketten, die eine 1,3-Dioxan-Gruppe aufweisen, dadurch gekennzeichnet, daß das Polymer der folgenden allgemeinen Formel genügt:

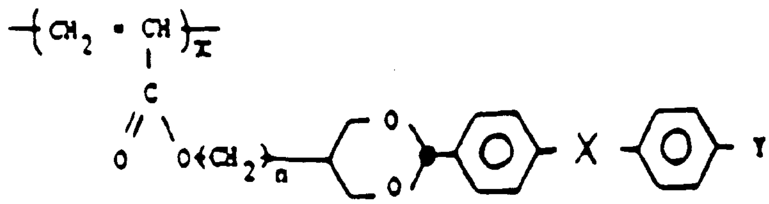

worin x den Polymerisationsgrad anzeigt,
X die Gruppe

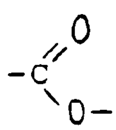 oder 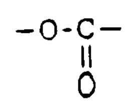 ,

Y die Grupe CN, $CF_3$ oder $OCH_3$ und
$2 \leqq n \leqq 12$ ist.

2. Mesomorphes Polymer nach Anspruch 1, dadurch gekennzeichnet, daß n den Wert 6 hat, X die Gruppe

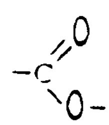

und Y die Gruppe $OCH_3$ ist.

3. Mesomorphes Polymer nach Anspruch 1, dadurch gekennzeichnet, daß n den Wert 6 hat, X die Gruppe

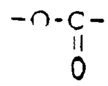

und Y die Gruppe $OCH_3$ ist.

4. Mesomorphes Polymer nach Anspruch 1, dadurch gekennzeichnet, daß n den Wert 6 hat, X die Gruppe

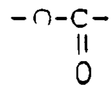

und Y die Gruppe $CF_3$ ist.

5. Verfahren zur Herstellung der mesomorphen Polymere gemäß einem der Ansprüche 1 bis 4, dadurch gekennzeichnet, daß das Verfahren die folgenden Stufen umfaßt:

– 1. Stufe: ein ω-Bromalkanol wird durch Reduktion der entsprechenden ω-Bromalkansäure erhalten;

– 2. Stufe: ein α-(2-Tetrahydropyranoxy)-ω-bromalkan wird durch Umsetzung des in der ersten Stufe erhaltenen ω-Bromalkohols mit Dihydropyran in Gegenwart eines Sulfonharzes hergestellt;

– 3. Stufe: ein (2-Tetrahydro-ω-pyranoxyalkyl)malonsäureethylester wird durch Reaktion zwischen dem Produkt aus der 2. Stufe und Natriumethylmalonat erhalten;

– 4. Stufe: ein ω-(2-Tetrahydropyranoxy)-2-hydroxymethylalkanol wird durch Reduktion des in der 3. Stufe erhaltenen Produktes hergestellt;

– 5. Stufe: ein (2-Hydroxymethyl)-α,ω-alkandiol wird durch Demaskierung der Hydroxylgruppe des in der 4. Stufe erhaltenen Produktes hergestellt;

– 6. Stufe: Verbindungen der allgemeinen Formel

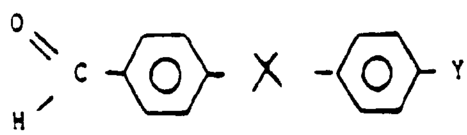

werden hergestellt durch Veresterung entweder des

. 4-Carboxybenzaldehyds mit dem in 4-Stellung durch den Rest Y substituierten Phenol, falls X die Gruppe

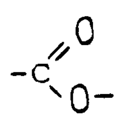

. ist, oder durch Veresterung des 4-Hydroxybenzaldehyds mit der in 4-Stellung durch den Rest Y substituierten Benzoesäure, falls X die Gruppe

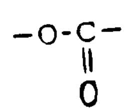

ist;

– 7. Stufe: eine Verbindung der allgemeinen Formel

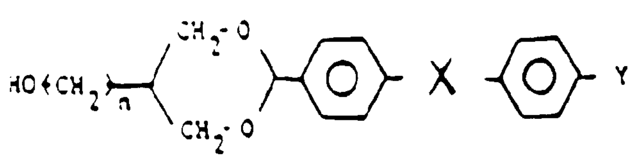

wird durch Acetalisierung der in der 6. Stufe erhaltenen Verbindung mit dem (2-Hydroxymethyl)-α,ω-alkandiol, welches in der 5. Stufe erhalten wurde, hergestellt;
– 8. Stufe: ein Monomer der allgemeinen Formel:

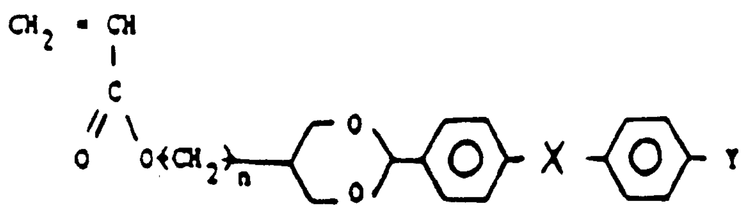

wird durch Verestern der in der 7. Stufe erhaltenen Verbindung mit Acrylsäurechlorid hergestellt;
– 9. Stufe: das trans-Isomere des in der 8. Stufe erhaltenen Monomers wird polymerisiert.